# EUROPEAN PATENT SPECIFICATION

(11) **EP 2 061 546 B1**
(45) Date of publication and mention of the grant of the patent: **11.06.2014**
(21) Application number: 07841512.2
(22) Date of filing: 29.08.2007
(51) Int. Cl.: A61M 25/09

(54) **LOOP TIP WIRE GUIDE**
FÜHRUNGSDRAHT MIT SCHLEIFENSPITZE
GUIDE-FIL À EXTRÉMITÉ EN BOUCLE

(30) Priority: 07.09.2006 US 842827 P
(43) Date of publication of application: 27.05.2009
(73) Proprietor: Cook Medical Technologies LLC, Bloomington, IN 47404 (US)
(72) Inventor: CARTER, Matthew, P., Dobson, NC 27017 (US); AGNEW, Charles, W., West Lafayette, IN 47906 (US)
(74) Representative: Garratt, Peter Douglas
(86) International application number: PCT/US2007/077072
(87) International publication number: WO 2008/030737

(56) References cited:
- WO-A-2006/039217
- FR-A- 2 625 437
- US-A- 5 728 122

## Description

### BACKGROUND

### Technical Field

This invention relates to wire guides used in the placement of medical devices, and more particularly to wire guides having a loop tip.

### 2. Background Information

Wire guides are elongate flexible members used to provide a path along which another medical device can be moved. The path provided by the wire guide can be used to navigate another medical device, such as a catheter through a body vessel. The use of wire guides to define such a path is known in the art. Briefly, a wire guide is navigated through a body lumen toward a point of treatment. Once positioned within the lumen, a therapeutic or diagnostic device, (i.e., a catheter) may be advanced over the wire guide to the target site and the desired therapeutic or diagnostic steps may be performed. The wire guide provides an established path for placing other devices and eliminates the need for performing delicate navigation procedures for each device passed into the body lumen, for example when additional procedures are performed. In some procedures, it is desirable to be able to withdraw the wire guide back through the catheter.

During placement of the wire guide, an operator must navigate the wire guide through a tortuous pathway in the body lumen due to the presence of natural bends and/or curves, or unnatural impediments, such as tumors, build-ups, and /or strictures. The presence of a tortuous path may make navigation of a wire guide through the path difficult, for example, the tip of the wire guide may get bent away from the desired path or caught in a stricture, or in some cases even perforate the wall of the lumen, etc. making further navigation into the lumen difficult or impossible.

The prior art contains many examples of wire guides having straight, flexible tips intended to aid in navigation of tortuous body lumens. The presence of a straight tip, however, may make navigation more difficult. For example, upon encountering an impediment, the straight tip may bend (reflex) into the lumen wall and become caught. Contact of the straight tip with the lumen wall may prevent the wire guide from advancing further into the lumen as well as possibly damaging the lumen wall. The prior art also contains examples of wire guides having loop tips as shown for example in WO 2006/039217.

What is needed is an improved guide wire tip for navigating a tortuous body lumen and which addresses the other deficiencies described above, but which is still readily retractable into a catheter.

### BRIEF SUMMARY

The present invention is directed to a wire guide having a loop tip that provides significant improvements and advantages over conventional straight wire guide tips. The scope of the invention is set forth in the appended claims.

According to one aspect, a wire guide is provided. The wire guide includes an elongate shaft having a proximal portion and a distal portion. The distal portion of the shaft includes a loop that is releasably connected to the shaft in a first looped configuration. The loop is disconectable at a detachment point to form a a second configuration wherein the loop is at least partially disconnected from the shaft.

According to another aspect, a medical system is provided. The system includes a first elongate member having a lumen disposed at least partially through a distal portion of the first elongate member. The system further includes a second elongate member extending through at least a portion of the first elongate member. The second elongate member includes an elongate shaft having a proximal portion and a distal portion, the distal portion extending distally of the first elongate member. The distal portion of the shaft includes a loop portion releasably connected to a distal end portion of the shaft. The distal portion of the second elongate member is retractable into the first elongate member by contacting the distal end of the elongate shaft with an edge of the first elongate member to at least partially release the loop portion from the shaft.

In another aspect, a method of making a wire guide is provided. The method includes providing an elongate shaft having a proximal portion and a distal portion and providing a loop at the distal portion of the shaft. The method further includes releasably connecting the loop to the shaft to form a first configuration for the wire guide.

In another aspect, a method of disconnecting a loop portion of a medical device is provided. The method includes providing a first elongate member having a lumen at least partially extending therethrough and providing a second elongate member having a shaft and a distal end portion forming a loop portion. The loop portion extends distally from the second elongate member and the second elongate member includes a proximal portion at least partially extending through the lumen. The method further includes retracting the second elongate member proximally into the lumen to at least partially disconnect the loop from the shaft.

The foregoing paragraphs have been provided by way of general introduction, and are not intended to limit the scope of the following claims. The presently preferred embodiments, together with further advantages will be best understood by reference to the following detailed description taken in conjunction with the accompanying drawings.

### BRIEF DESCRIPTION OF THE DRAWINGS

Embodiments will now be described by way of example with reference to the accompanying drawings, in which:
FIG. 1A is a partial side view of a wire guide having a loop tip;
FIG. 1B is a partial side view of an alternative connection for the loop tip shown in FIG. IA;
FIG. 1C is a partial side view of an alternative connection for the loop tip shown in FIG. 1A;
FIG. 1D is a cross-sectional view of a connecting region of the loop shown in FIG. 1A;
FIG. 1E is a cross-sectional view of an alternative connecting region of the loop shown in FIG. 1A;
FIG. 2A is a partial side view of the wire guide shown in FIG. 1 partially disposed in a catheter, with the wire guide in a first configuration;
FIG. 2B is a partial side view of the wire guide and catheter shown in FIG. 2A, showing the wire guide in a second configuration with the loop unconnected;
FIG. 2C is a partial side view of the wire guide and catheter shown in FIG. 2B with the wire guide withdrawn into the catheter;
FIG. 2D is a partial side view of the wire guide and catheter shown in FIG. 2C with the wire guide re-extended from the catheter and forming the loop tip;
FIGS. 3A-3E illustrate cross-sectional views of the distal portion forming the loop of the wire guide;
FIG. 4A is a partial side view of an alternative embodiment of the wire guide;
FIG. 4B is a partial side view of an alternative embodiment of the loop of the wire guide shown in FIG. 4A;
FIG. 5 A is a partial side view of the wire guide shown in FIG. 4A partially disposed in a catheter, with the wire guide in a first configuration;
FIG. 5B is a partial side view of the wire guide and catheter shown in FIG. 5A with the wire guide in the second configuration;
FIG. 5C is a partial side view of the wire guide and catheter shown in FIG. 5B with the wire guide re-extended and the loop removed;
FIG. 6A is a partial side view of the wire guide having an alternative detachment portion partially disposed in a catheter, with the wire guide in a first configuration;
FIG. 6B is a partial side view of the wire guide and catheter shown in FIG. 6A with the wire guide in the second configuration;
FIG. 7 is a side view of a catheter comprising a handle with a wire guide disposed through a lumen thereof;
FIG. 8 illustrates the wire guide of the present invention entering the common bile duct;
FIG. 9 is a side view illustrating a wire guide entering a tortuous path within a body vessel;
FIGS. 10A- 10G illustrate alternative loop configurations for the embodiments shown in FIGS IA-1C; and
FIGS. 11A-11G illustrate alternative loop configuration for the embodiments shown in FIGS. 4 A and 4B.

### DETAILED DESCRIPTION OF THE INVENTION

The invention is described with reference to the drawings in which like elements are referred to by like numerals. The relationship and functioning of the various elements of this invention are better understood by the following detailed description. However, the embodiments of this invention as described below are by way of example only, and the invention is not limited to the embodiments illustrated in the drawings. It should also be understood that the drawings are not to scale and in certain instances details have been omitted, which are not necessary for an understanding of the present invention, such as conventional details of fabrication and assembly.

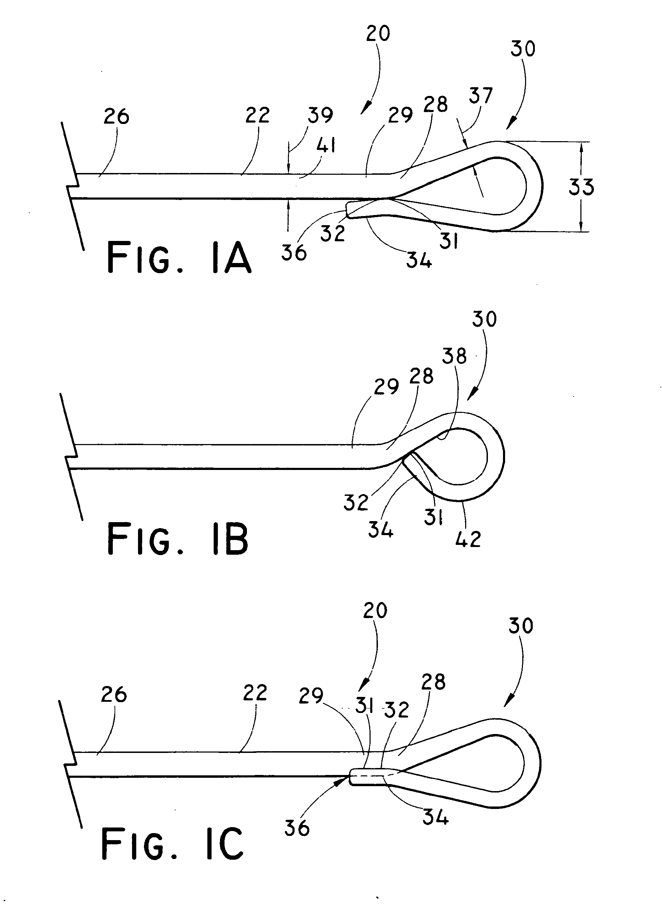

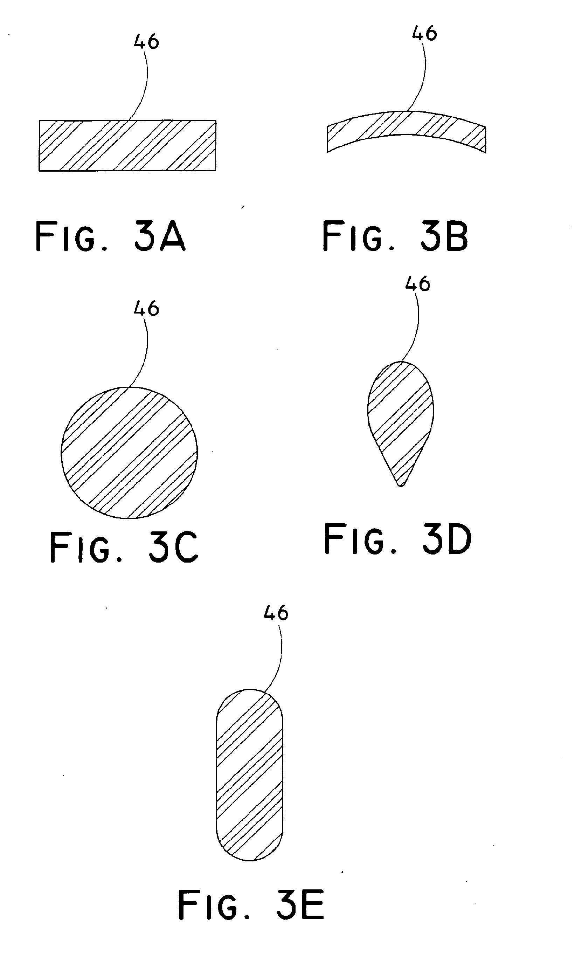

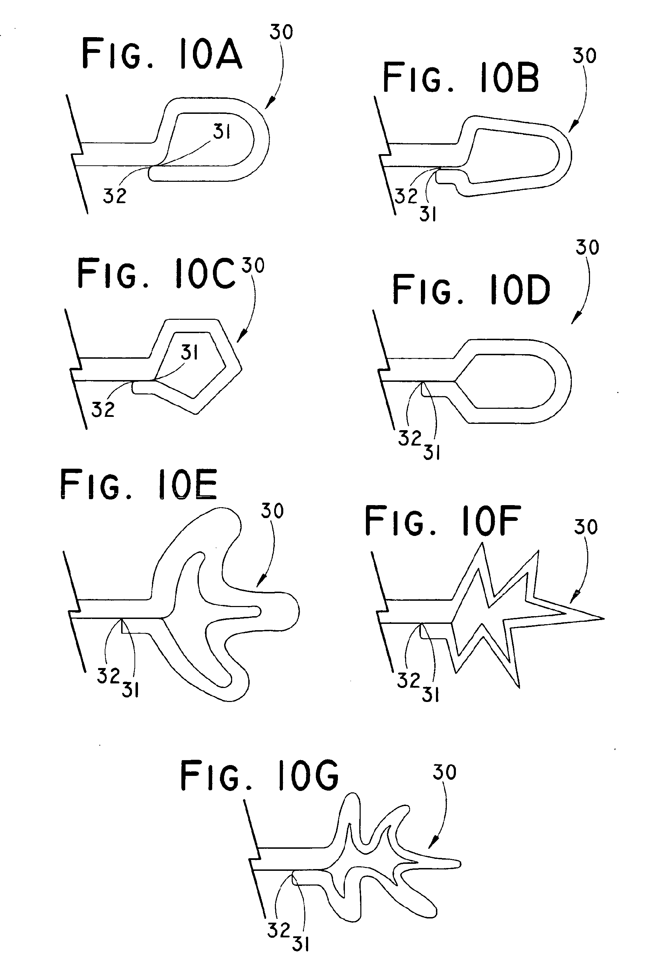

FIGS. 1A-1C illustrate a wire guide 20. The wire guide 20 includes an elongate shaft 22 having a proximal portion 26 and a distal portion 28 having an intermediate portion 29. The distal portion 28 may be formed into a loop 30 by folding the distal portion 28 back onto itself. A connecting region 32 is formed where an end portion 34 of the distal portion 28 contacts the intermediate portion 29. As shown, the loop 30 is generally curvilinear in shape, although other forms are possible. In some embodiments, the loop is generally oval in shape. Additional shapes include, but are not limited to round, arched, and parabolic. See FIGS. 10A-10G for examples of additional configurations that are possible for the loop 30. The cross-sectional shape of the distal portion 28 or portions thereof, may be any shape. For example, the cross-sectional shape of the distal portion 28 may be rectangular, arched rectangular, circular, teardrop, oval and the like. See FIGS. 3A to 3E showing cross section 46 of the distal portion 28. The thickness of the distal portion 28 may be varied to provide flexibility for the loop 30 as will be understood by one skilled in the art. The loop 30 may be deformable as the loop 30 moves through a tortuous body lumen and the shape of the loop 30 refers to the general shape in a pre-advancement state. The loop 30 has a diameter 33 measured at the widest portion of the loop 30.

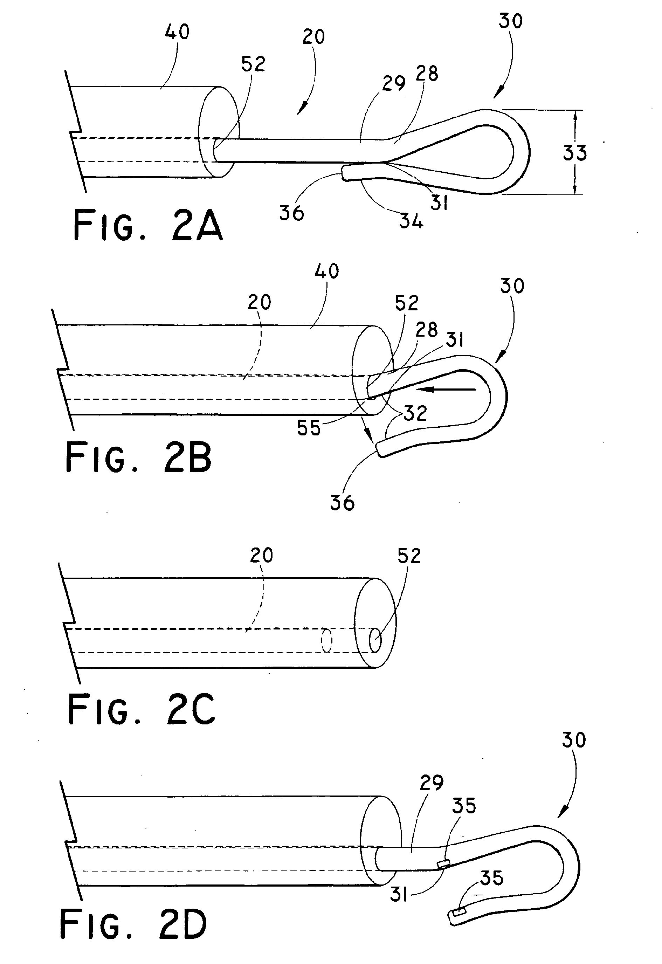

As shown in FIGS. 1A-1C, the connecting region 32 is formed where the end portion 34 meets the intermediate portion 29 of the distal portion 28 of the wire guide 20. A detachment point 31 may be formed where the end portion 34 meets the intermediate portion 29 as shown in FIGS. 1A-1C. In some embodiments, the detachment point may be located apart from the loop portion as discussed in detail below. The connecting region 32 may be any length as long as the connection formed (as described below) is sufficient to maintain the connection during advancement of the wire guide 20 through the tortuous body lumen and allow for release of the connection at the detachment point 31 when the wire guide 20 is withdrawn into a catheter 40 (see FIGS. 2A-2D). As shown in the embodiment illustrated in FIG. 1C, the end portion 34 may include an end 36 that protrudes proximally from the connecting region 32 and that may be used to contact an apparatus, such as the catheter 40, to facilitate release of the detachment point 31 at the connection between the end portion 34 and the intermediate portion 29 (See FIGS. 2A and 2B). Alternatively, the end portion 34 of the distal portion 28 may abut an interior surface 38 of the loop 30 at the contacting region 32 to form a smooth profile for the loop 30 as shown in FIG. 1B. A portion of an exterior surface 42 of the loop 30 may be used to contact an apparatus to facilitate release of the detachment point 31 at the connecting region 32. In some embodiments, the end 36 may be curved or wrapped around a portion of the intermediate portion 29 of the distal portion 28 to provide a greater surface area for the contacting region 32 and to decrease the protrusion of the end 36 from the shaft 22 as shown in FIG. 1C. In some embodiments, the connection at the detachment point 31 between the end portion 34 of the distal portion 28 may be removed by contacting the end 36 or the exterior surface 42 or both.

Any method may be used to form the removable connection at the detachment point 31 between the end portion 34 and the intermediate portion 29 of the wire guide 20. For example, the connection may be formed by bonding, including, but not limited to adhesive bonds and solder bonds, welding and molding. Combinations of these methods may also be used. One skilled in the art can control these methods, or combinations thereof, to form a detachment point 31 at the connecting region 32 that remains connected for navigation to the desired body lumen site and then is detachable at the detachment point 31 to facilitate withdrawal of the wire guide 20 into a catheter lumen 52 (see FIG. 2C) having a smaller diameter than the loop diameter 33. In some embodiments, the detachment at the detachment point 31 may be facilitated by physical force against another object such as the catheter 40 as described below. In other embodiments, the detachment of the connection may be by dissolution of a material that dissolves at a controlled rate as described below. In addition, in some embodiments, the connection may be covered with a coating that is dissolvable in solution, for example when exposed to blood, to further facilitate control of the separation of the connection. In some embodiments, the end portion 34 may also be connected using a suture, coil or other material to removably connect the end portion 34 to the intermediate portion 29. The suture, coil or other material may be dissolvable after a period of time to release the connection.

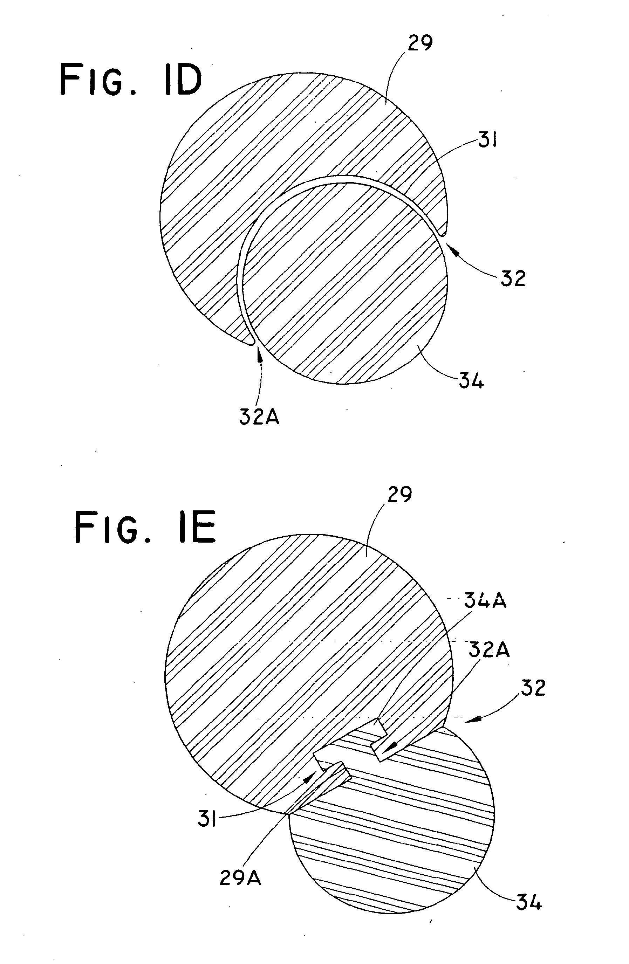

In some embodiments, the connection may be formed by a channel 32A formed at the connecting region 32. The channel 32A may be any type of interlocking engagement between the intermediate region 29 and the end portion 34. For example, as shown in FIG. 1D, the end portion 34 may slidaby fit into the channel 32A formed in the intermediate portion 29. The end portion 34 may be released from the channel 32A along the detachment point 31 by physical force against another object such as the catheter 40 as described below. The channel 32A may also be a lock and key configuration as shown in FIG. 1E where the intermediate portion 29 includes a receptacle 29A that is correspondingly sized and shaped to releasably interlock with a protrusion 34A on the end portion 34. Alternatively, the intermediate portion 29 may include a protrusion to releasably interlock with a receptacle on the end portion 34. The channel 32A may be a onc-way channel wherein the channel is a locking channel as the loop 30 is advanced through the lumen so that the loop 30 remains connected and an unlocking channel as the loop 30 is withdrawn into the catheter 40 so that the loop may be released at the detachment point 31 by sliding out of the channel.

In some embodiments, the end portion 34 may be magnetically connected to the intermediate portion 29 wherein one or more magnets 35 are separable with physical force or by demagnetizing the connection at the detachment point 31. An exemplary connection using magnets 35 is shown in FIG 2D, where one magnet 35 is located at the intermediate portion 29 and another magnet 35 is located at the end portion 34. A single magnet 35 may also be located at either the intermediate portion 29 or the end portion 34 and used to connect the loop 30 formed from a material that attracts a magnet, for example, steel. The magnet 35 may be a rare earth magnet or any other kind of magnet known to one skilled in the art.

The strength of the detachable connection at the detachment point 31 and the force required to release the end portion 34 from intermediate portion 29 and the distal portion 28 may be controlled as will be understood by one skilled in the art. In some embodiments, the strength of the connection and release of the connection at the detachment point may be controlled by selection of the process used to form the releasable connection. For example, in bonding, welding or molding, the strength and separability of the connection may be controlled by, but not limited to, the following: (i) addition of fillers and additives in the materials used to form the connection such as curing agents; accelerators; antioxidants; impact modifiers; lubricants; glass fillers; PTFE fillers; colorants; antistatics; plasticizers; minerals; cellulose; dielectrics; carbon fiber; metal oxides; graphite; and, any other moieties that may be mixed or combined as either in-chain or as pendant functional groups; (ii) treating the surfaces of the intermediate portion 29 and the end portion 34 prior to formation of the connection; (iii) selection of a coating that will serve as a bonding surface; (iv) selection of the size and geometry of a connection to, for example, control the size of the connected area upon which stress is concentrated or to control the stress distribution at the connecting region 32; (v) selection of materials with different coefficients of expansion to induce stress in the connecting region 32; and (vi) combinations thereof. The strength of the connection may also be controlled by the length of the connecting region 32 and therefore the amount of surface area available for contact, i.e. the shorter the connecting region 32, the more easily separable the connecting region 32 at the detachment point 31. In some embodiments, the connecting region may be about 0.01 and 0.040 inches (about 0.25-1 mm) in length.

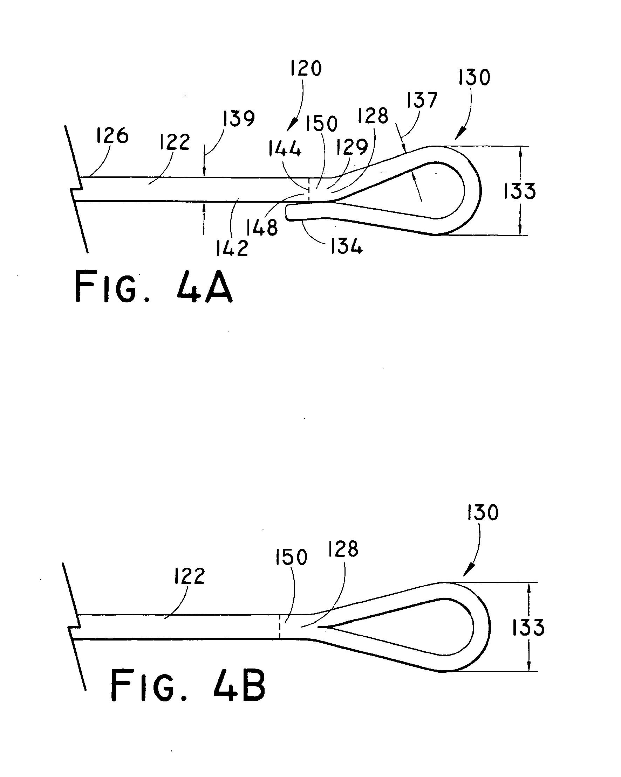

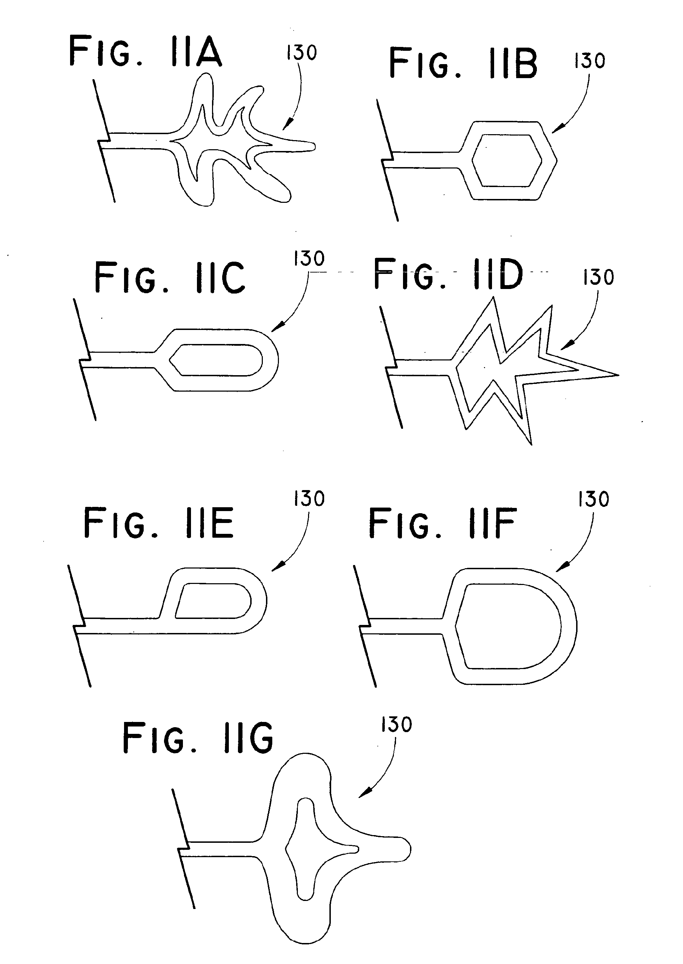

FIGS. 4A and 4B illustrate an alternative embodiment of a wire guide 120 according to the present invention. The wire guide 120 includes an elongate shaft 122 having a proximal portion 126 and a distal portion 128 having an intermediate region 129. The distal portion 128 may be formed into a loop 130 by folding the distal portion 128 back onto itself. When the loop 130 is formed by folding back onto itself, an end portion 134 of the distal portion 128 contacts the intermediate portion 129 to close the loop 130 as shown in FIG. 4A. The end portion 134 may extend out from the shaft 122 as shown in FIG. 4A, or the end portion 134 may connect with the intermediate portion 129 to form a smooth profile, similar to the profile shown in FIG. 1B. Alternatively, and, as shown in FIG. 4B, the loop 130 may be formed as a unitary structure where the loop 130 does not include a separate end that must be connected to the distal portion 128 to form the loop 130. In some embodiments, the loop 130 may be formed using laser cutting techniques as are known to those skilled in the art. The loop 130 may be generally curvilinearly-shaped, similar to the loop 30 described above, and has a diameter 133 at the widest portion of the loop 130. See FIGS. 11A-11G for examples of additional configurations that are possible for the loop 130.

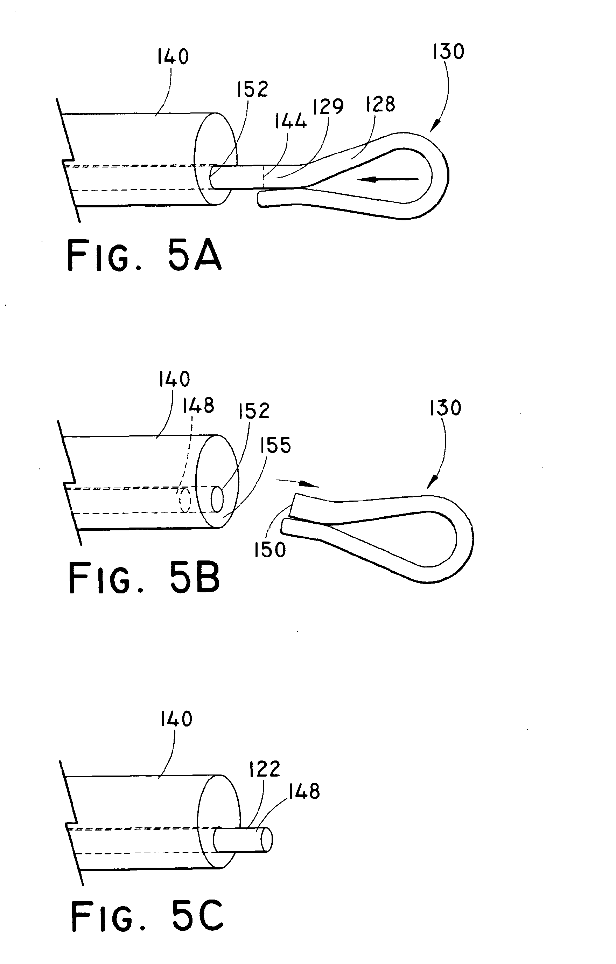

The wire guide 120 further includes a detachment point 144 between a shaft detachment portion 148 and a distal detachment portion 150 on the distal portion 128. The distal portion 128 including the loop 130 may be removably connected to the shaft 122 of the wire guide 120. The loop 130 may be separated from the shaft 122 of the wire guide 120 at the detachment point 144 to allow for the shaft 122 to be withdrawn into a catheter 140 (see FIGS. 5A-5C). The connection between the distal portion 128 and the shaft 122 is sufficient to allow the loop 130 to remain connected to the shaft 122 during advancement of the wire guide 120 through the body lumen to the desired location and to allow detachment of the loop 130 from the shaft 122 when the shaft 122 is retracted into the catheter 140.

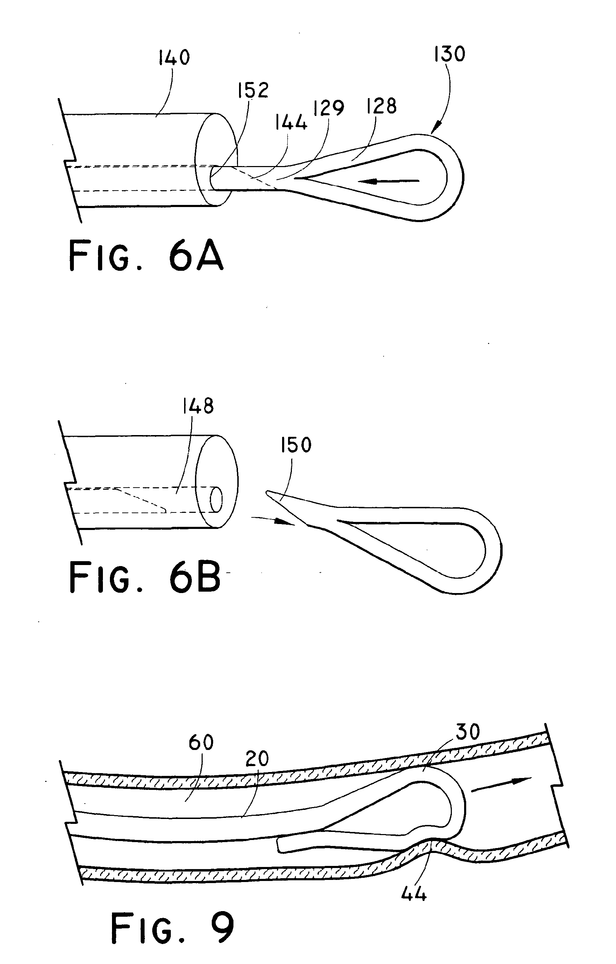

The shaft detachment portion 148 and the distal detachment portion 150 may be shaped to compliment each other in the connected configuration to form a smooth outer surface 142 of the shaft 122. In addition, the shaft detachment portion 148 may be dome shaped and the distal detachment portion 150 may be curved inwardly (concaved) to nest with the dome shape of the shaft detachment portion 148 as shown in FIG. 5B. In some embodiments, the detachment point 144 may be axially elongated, extending along the shaft 122 to provide a larger surface area for connection between the shaft detachment portion 148 and the distal detachment portion 150 as shown in FIGS. 6A and 6B. The shaft detachment portion 148 may be rounded at the end to facilitate forward movement of the shaft 122 after the loop 130 is removed. Alternative shapes and configurations may be used for the shaft detachment portion 148, the distal detachment portion 150 and the detachment point 144.

Any method may be used to form the removable connection at the detachment point 144 between the shaft detachment portion 148 and the distal detachment portion 150 of the wire guide 120. For example, methods similar to the methods described above for the connecting region 32 described above may be used. The strength of the connection at the detachment region may be controlled similar to the methods described above.

Any suitable material can be used for the wire guide 20, 120 and portions thereof. The material chosen need only be biocompatible, or made biocompatible, and able to be formed into the structures described herein. Examples of suitable materials include, but are not limited to stainless steel, tantalum, nitinol; gold, silver, tungsten, platinum, inconel, cobalt-chromium alloys and iridium, all of which are commercially available metals or alloys used in the fabrication of medical devices. Portions of the wire guide may be formed from a medically-acceptable polymer. For example, exemplary polymers include, but are not limited to, cellulose acetate, cellulose nitrate, silicone, polyethylene, high density polyethylene, polyethylene teraphthalate, polyurethane, polytetrafluoroethylene, polyamide, polyester, polyorthoester, polyvinyl chloride (PVC), polypropylene, acrylonitrile-butadiene-styrene (ABS), polycarbonate, polyurethane, nylon silicone, and polyanhydride.

Portions of the wire guide 20, 120 may also be made from a bioabsorbable material. For example, the distal portion 128 of the wire guide 120 including the loop 130 may be bioabsorbable so when the distal portion is removed from the shaft 122, the distal portion 128 degrades in the gastrointestinal tract as described below. A number of bioabsorbable homopolymers, copolymers, or blends of bioabsorbable polymers are known in the medical arts. These include, but are not necessarily limited to, polyesters including poly-alpha hydroxy and poly-beta hydroxy polyesters, polycaprolactone, polyglycolic acid, polyetheresters, poly(p-dioxanone), polyoxaesters; polyphosphazenes; polyanhydrides; polycarbonates including polytrimethylene carbonate and poly(iminocarbonate); polyesteramides; polyurethanes; polyisocyantes; polyphosphazines; polyethers including polyglycols polyorthoesters; expoxy polymers including polyethylene oxide; polysaccharides including cellulose, chitin, dextran, starch, hydroxyethyl starch, polygluconate, hyaluronic acid; polyamides including polyamino acids, polyester-amides, polyglutamic acid, poly-lysine, gelatin, fibrin, fibrinogen, casein, and collagen.

In some embodiments, the wire guide 20, 120 or portions thereof, may comprise one or more metallic bioabsorbable materials. Suitable metallic bioabsorbable materials include magnesium, titanium, zirconium, niobium, tantalum, zinc and silicon and mixtures and alloys. For example, a zinc-titanium alloy such as discussed in U.S. Patent 6,287,332 to Bolz et al. can be used. The metallic bioabsorbable material can further contain lithium, sodium, potassium, calcium, iron and manganese or mixtures thereof. For example, an alloy containing lithium:magnesium or sodium:magnesium can be used. The physical properties of the frame can be controlled by the selection of the metallic bioabsorbable material, or by forming alloys of two or more metallic bioabsorbable materials. For example, when 0.1% to 1%, percentage by weight, titanium is added to zinc, the brittle quality of crystalline zinc can be reduced. In another embodiment, when 0.1% to 2%, percentage by weight, gold is added to a zinc-titanium alloy, the grain size of the material is reduced upon curing and the tensile strength of the material increases.

The wire guide 20, 120, or portions thereof, may comprise a wire, a tubular member or a sheet of material. Further, the wire guide 20, 120 or portions thereof may be formed from a series of layers, or as a coated core structure. For example, in one embodiment, the shaft 22, 122 may comprise a nitinol core with a polytetrafluoroethylene (PTFE) covering. The loop 30 may also be formed of nitinol and may include a PTFE covering. When present, the covering should not interfere with the removable connection between either the end portion 34 and the distal portion 28 or the shaft detachment portion 148 and the distal detachment portion 150.

A variety of shapes and sizes of shafts 22, 122 and loops 30, 130 may be used, and these can both be optimized based on particular applications. The dimensions of the shaft 22, 122 and the loop 30, 130 will depend upon various factors, including the intended use of the wire guide 20, 120 and the vessels into which the wire guide 20, 120 will be positioned. For a wire guide 20, 120 intended to cannulate the common bile duct, suitable dimensions include a shaft diameter 39, 139 of between approximately 0,4 mm (0.016 inches) and approximately 1 mm (0.038 inches), and preferably comprises a diameter 39, 139 of approximately 0,889 mm (0.035 inches). The distal portion diameter 37, 137 forming the loop 30, 130 of the wire guide 20, 120 preferably has a diameter of between approximately 0,07 mm (0.003 inches) and approximately 0,25 mm (0.010 inches), and preferably comprises a diameter of approximately 0,14 mm (0.006 inches). When the loop 30, 130 is ovoid in shape and delivered to the bile duct, the length of the loop 30, 130 may be between approximately 4 and approximately 5 millimeters, and the width 33, 133 at the widest portion of the loop 30, 130 may be between approximately 2 and approximately 3 millimeters. One skilled in the art will recognize that other sizes and shapes are possible depending on the bodily location the wire guide 20, 120 is configured to enter. For example, the loop 30, 130 may also be configured to enter the colon, pancreas and esophagus that may require different sizes than described above. Any size and shape loop 30, 130 may be used with the present invention.

As discussed above, a dissolvable coating may be provided over the detachment point 31, the connecting region 32 and the detachment point 144. Additional coatings may also be applied to at least a portion of the wire guide 20, 120. The coating(s) may be applied by dipping, molding or spraying a suitable coating material, such as PTFE, urethane and/or other polymeric coatings, directly to the wire guide 20, 120 or portions thereof. Any coating applied to the wire guide 20, 120 will be applied so that the coating will not interfere with the removable connection on the wire guide 20, 120.

In some embodiments, a thin heat shrinkable material may be used for the coating, such as PTFE. The heat shrinkable material facilitates manufacturing while providing a lubricious coating, which facilitates navigation. In preferred embodiments, the thickness of the coating is between approximately 0,025 mm and 0,25 mm (0.001 and 0.010 inches). In particularly preferred embodiments, the thickness of the coating is between approximately 0,025 and 0,127 mm (0.001 and 0.005 inches). In still more preferred embodiments, the thickness of the coating is between approximately 0.001 and 0.002 inches. These preferred thicknesses provide suitable coatings while not adding significantly to the overall thickness of the device.

Also, the wire guide 20, 120 or portions thereof, with or without the coating described above, may be treated with a hydrophilic coating or hybrid polymer mixture, such as those based on polyvinyl puroladine and cellulose esters in organic solvent solutions. These solutions make the wire guide particularly lubricious when in contact with body fluids, which aids in navigation.

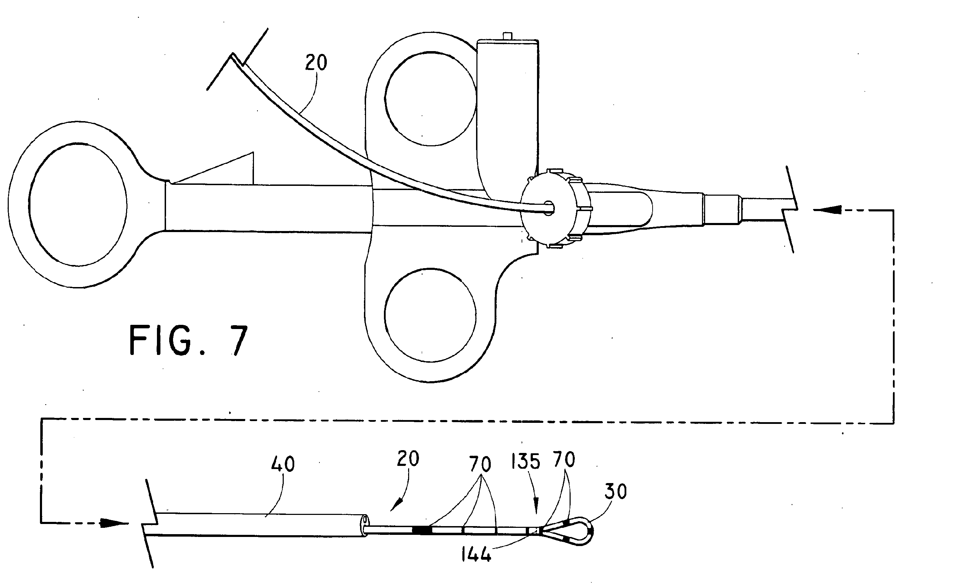

Radiopaque materials may be added in the coating. Also, radiopaque materials known in the art may be placed directly on or in the shaft 22, 122 and the loop 30, 130 and other portions of the wire guide 20, 120. For example, radiopaque materials may be placed on both sides of the connection region 32 and the detachment region 135 so that separation at the detachment point 31 and the detachment point 144 may be viewable by the wire guide operator. For example, a plurality of bands 70 may be present on the wire guide 20, 120 and/or the catheter 40, 140 as shown in FIG. 7.

Several examples of suitable radiopaque materials and markers are known in the art, and any suitable material and/or marker can be utilized in the present invention. Common radiopaque materials include barium sulfate, bismuth subcarbonate, and zirconium dioxide. Other radiopaque elements include: cadmium, tungsten, gold, tantalum, bismuth, platinum, iridium, and rhodium. In one embodiment, iodine may be employed for its radiopacity and antimicrobial properties. Radiopacity is typically determined by fluoroscope or x-ray film. Radiopaque, physiologically compatible materials include metals and alloys selected from the Platinum Group metals, especially platinum, rhodium, palladium, rhenium, as well as tungsten, gold, silver, tantalum, and alloys of these metals. These metals have significant radiopacity and in their alloys may be tailored to accomplish an appropriate blend of flexibility and stiffness. They are also largely biocompatible. For example, a platinum/tungsten alloy, e.g., 8% tungsten and the remainder platinum may be used.

Operation of the wire guide 20, 120 of the present invention is as follows. The wire guide 20, 120 may be provided to the operator preassembled with the wire guide 20, 120 loaded into a catheter, such as the catheter 40, 140. The catheter may be any catheter known to one skilled in the art, including, but not limited to, multi-lumen catheters, balloon catheters, stent delivery catheters, cannulae, papillotomes and sphincterotomes, and the like. In some embodiments, the wire guide 20, 120 may be back loaded into the lumen 52, 152 so that the distal portion 28, 128 including the loop 30, 130 of the wire guide 20, 120 extends distally from the catheter 40, 140. Back loading refers to introduction of the proximal portion 26, 126 of the wire guide 20, 120 into the distal end of the catheter 40, 140 until the proximal portion 26, 126 extends out of a proximal wire guide port near the proximal end of the catheter 40, 140 (not shown). The wire guide 20, 120 may be oriented in any direction when assembled into the catheter 40, 140. For example, when the wire guide 20, 120 is back loaded into a catheter 40, 140 having an offset lumen, the wire guide 20, 120 may be oriented so that the loop 30, 130 is generally centered with respect to the catheter 40, 140.

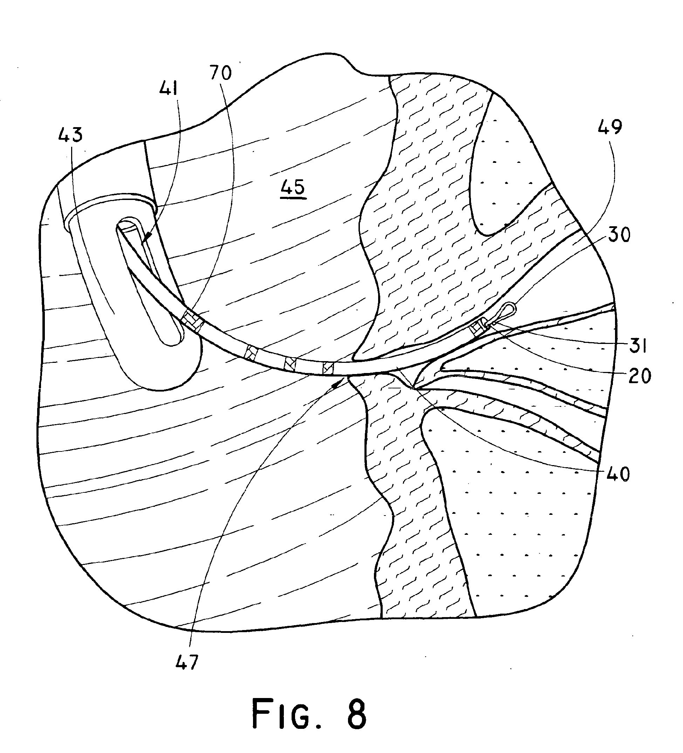

The wire guide 20, 120 may be advanced through the tortuous body lumen to the desired location in the first looped configuration. The catheter 40, 140 may then be advanced over the wire guide 20, 120, either simultaneously with or subsequent to, following standard procedures known to one skilled in the art. FIG. 8 illustrates the wire guide 20 extending out an opening 41 of an endoscope 43. The endoscope 43 is positioned in the duodenum 45 and the wire guide 20 is shown entering the biliary tree 49 through the ampullary orifice (Papilla of Vater) 47. FIG. 9 is an enlargement of the wire guide 20 in the first configuration moving through a torturous path 44 within a body vessel 60. As illustrated in FIG. 9, the loop 30 deforms slightly in response to the torturous path 44. This allows the wire guide 20 to continue navigating along the interior of the body vessel 60. The catheter 40, 140 may be introduced over the wire guide 20, 120 to the treatment location. In addition to deforming as the wire guide 20 navigates the body vessel 60, the loop 30 allows the wire guide 20 to enter through a narrow stricture, such as a sphincter, without the wire guide 20 getting stuck in the tissue around the narrow stricture. The loop 30 may be self-centering with respect to the shaft 22 of the wire guide 20 and rigid enough so that the wire guide 20 may be pushed though the narrow stricture using the loop 30 to atraumatically cannulate the stricture.

Once the catheter 40, 140 reaches the desired location, the operator may wish to retract the wire guide 20, 120 into the catheter 40, 140 for removal of the wire guide 20, 120 or for temporary retraction and re-extension. Retraction and re-extension of the wire guide 20 is illustrated in FIGS. 2A-2D. As shown in FIG. 2A, the wire guide 20 is in the first configuration and the width 33 of the loop 30 is greater than the diameter of the lumen 52. In FIG. 2B, the end portion 34 is disconnected from the distal portion 28 at the connecting region 32, thus releasing the end 36 of the loop 30 to form the second configuration wherein the wire guide 20 is straightenable or straight for retraction into the catheter. The connection between the end portion 34 and the distal portion 28 at the detachment point 31 may be released by any method. For example, the wire guide 20 may be retracted into the catheter 40 so that the end 36 may be pulled against an edge 55 of the catheter 40 to disconnect the connection of the end portion 34 from the distal portion 28 at the detachment point 31. Once the connection is released, the wire guide 20 may be completely retracted into the catheter 40 as shown in FIG. 2C. The loop 30 of the wire guide 20 of this embodiment is flexible enough to be unbent (straightened) and retracted into the catheter 40. In some embodiments, for example when a shape memory metal is used to form a portion of the loop 30, the wire guide 20 may be adapted to reform or bend back into the shape of the loop 30 when the wire guide 20 is re-extended from the catheter 40 as shown in FIG. 2D even though the connection between the end portion 34 and the distal portion 28 has been severed. In some embodiments, the connection between the end portion 34 and the distal portion 28 at the connecting region 32 may be disconnected by dissolution of a connecting material after a period of time. In some embodiments, a combination of methods may be used.

Similarly, the retraction and re-extension of the wire guide 120 is illustrated in FIGS. 5A-4C, 6A and 6B. As shown in FIG. 5A, the wire guide 120 is shown in the first looped configuration wherein the width 133 of the loop 130 is greater than the diameter of the lumen 152. In FIG. 5B, the loop 130 is disconnected at the detachment point 144 to form the second configuration so that the loop 130 is removed at the distal detachment portion 150 from the shaft detachment portion 148. The loop 130 may be disconnected from the shaft 122 by any method known to one skilled in the art. For example, the loop 130 may be pulled against an edge 155 of the catheter 140 as the wire guide 120 is retracted into the catheter 140 to disconnect the loop 130 from the shaft 122 of the wire guide 120. The shaft detachment portion 148 may then be retracted into the catheter 140. In some embodiments, the connection between the loop 130 and the shaft 122 occurs in the duodenum. In the duodenum, the loop 130, when formed from a bioabsorbable material, may be degraded. In addition, or alternatively, the loop 130 may be passed out of the body through the gastrointestinal tract. As shown in FIG. 5C, the shaft detachment portion 148 in the second configuration may be re-extended from the catheter 140 after disconnection of the loop 130. In some embodiments, shown in FIGS. 5B, 5C, and FIG. 6B, the shaft detachment portion 148 is curved at the end to provide for smoother passage through the body lumen when the shaft 122 is re-extended. The shaft detachment portion 148 and the distal detachment portion 150 may be separated from each other at the detachment point 144 by any method, for example, by physical force, by dissolution or combinations thereof, as described above.

Any other undisclosed or incidental details of the construction or composition of the various elements of the disclosed embodiment of the present invention are not believed to be critical to the achievement of the advantages of the present invention, so long as the elements possess the attributes needed for them to perform as disclosed. The selection of these and other details of construction are believed to be well within the ability of one of even rudimentary skills in this area, in view of the present disclosure. Illustrative embodiments of the present invention have been described in considerable detail for the purpose of disclosing a practical, operative structure whereby the invention may be practiced advantageously. The designs described herein are intended to be exemplary only. The novel characteristics of the invention may be incorporated in other structural forms without departing from the scope of the invention. Unless otherwise indicated, all ordinary words and terms used herein shall take their customary meaning as defined in The New Shorter Oxford English Dictionary, 1993 editi*on.* All technical terms shall take on their customary meaning as established by the appropriate technical discipline utilized by those normally skilled in that particular art area. All medical terms shall take their meaning as defined by Stedmun's Medical Dictionary, 27th editi*on.*

It is therefore intended that the foregoing detailed description be regarded as illustrative rather than limiting, and that it be understood that it is the following claims, including all equivalents, that are intended to define the scope of this invention.

## Claims

1. A wire guide 20 comprising:
an elongate shaft 22 including a proximal portion and a distal portion, the distal portion comprising a loop 30 releasably connected to the shaft in a first looped configuration, **characterised in that** the loop is disconnectable at a detachment point and forms a second configuration wherein the loop is at least partially disconnected from the shaft; and **in that**
an end portion of the loop is connected to the shaft by a channel connection 32, 32A or a magnetic connection 29, 34, 35.

2. The wire guide according to claim 1, wherein the loop is completely removable from the shaft in the second configuration.

3. The wire guide according to claims 1 or 2, wherein the distal portion further comprises an intermediate portion and an end portion, the end portion removably connected to the intermediate portion to form the loop.

4. The wire guide according to any of the preceding claims, wherein at least a portion of the wire guide comprises a bioabsorbable material.

5. The wire guide according to any of the preceding claims, wherein at least a portion of the wire guide comprises nitinol.

6. The wire guide according to any of the preceding claims, wherein at least a portion of the wire guide further comprises a coating.

7. The wire guide according to any of the preceding claims, wherein the connecting region comprises a radiopaque material.

8. The wire guide according to any of the preceding claims, wherein the wire guide is configured to be disposed in the gastrointestinal tract of a patient.

9. The wire guide according to any of the preceding claims, in combination with:
a first elongate member comprising a proximal portion and a distal portion, the first elongate member including a lumen disposed at least partially through the distal portion;
wherein an end portion of the loop is connected to the shaft by a channel connection or a magnetic connection; and
wherein the distal portion of the wire guide is retractable into the first elongate member by contacting the distal end of the elongate shaft with an edge of the first elongate member to at least partially release the loop from the shaft.

10. The wire guide according to claim 9, further comprises a connecting region for releasably connecting the loop portion to the shaft.

11. The wire guide according to claim 10, wherein the shaft comprises a shaft detachment portion that is retractable into the first elongate member and re-extendable from the first elongate member after the loop portion is removed from the shaft.

12. A method of disconnecting a loop portion of a medical device, the method comprising;
providing a first elongate member having a lumen at least partially extending therethrough;
providing a second elongate member having a shaft and a distal end portion forming a loop portion, the loop portion extending distally from the second elongate member and the second elongate member having a proximal portion at least partially extending through the lumen,
providing a channel connection or a magnetic connection for releasably connecting an end portion of the loop to the shaft for forming the loop;
retracting the second elongate member proximally through the lumen to at least partially disconnect the loop from the shaft.

13. The method according to claim 12 comprising contacting the distal end portion of the second elongate member with an edge on the distal portion of the first elongate member to disconnect the loop.

## Patentansprüche

1. Führungsdraht 20, umfassend:
ein langgestreckter Schaft 22 mit einem proximalen Abschnitt und einem distalen Abschnitt, wobei der distale Abschnitt eine Schleife 30 umfasst, die in einer ersten Schleifenkonfiguration lösbar mit dem Schaft verbunden ist, **dadurch gekennzeichnet, dass** die Schleife an einem Ablösepunkt gelöst werden kann und eine zweite Konfiguration bildet, in der die Schleife zumindest teilweise von dem Schaft gelöst ist; und dass ein Endabschnitt der Schleife über eine Kanalverbindung 32, 32A oder eine Magnetverbindung 29, 34, 35 mit dem Schaft verbunden ist.

2. Führungsdraht nach Anspruch 1, worin die Schleife in der zweiten Konfiguration vollständig von dem Schaft entfernt werden kann.

3. Führungsdraht nach den Ansprüchen 1 oder 2, worin der distale Abschnitt ferner einen Zwischenabschnitt und einen Endabschnitt umfasst, wobei der Endabschnitt abnehmbar mit dem Zwischenabschnitt der Schleife verbunden ist.

4. Führungsdraht nach einem der vorhergehenden Ansprüche, worin zumindest ein Teil des Führungsdrahts ein bioresorbierbares Material umfasst.

5. Führungsdraht nach einem der vorhergehenden Ansprüche, worin zumindest ein Teil des Führungsdrahts Nitinol umfasst.

6. Führungsdraht nach einem der vorhergehenden Ansprüche, worin zumindest ein Teil des Führungsdrahts ferner eine Beschichtung umfasst.

7. Führungsdraht nach einem der vorhergehenden Ansprüche, worin die Verbindungsregion ein röntgenopakes Material umfasst.

8. Führungsdraht nach einem der vorhergehenden Ansprüche, worin der Führungsdraht zur Anordnung in dem Magen-Darm-Trakt eines Patienten ausgelegt ist.

9. Führungsdraht nach einem der vorhergehenden Ansprüche, in Kombination mit:
einem ersten langgestreckten Element mit einem proximalen Abschnitt und einem distalen Abschnitt, wobei das erste langgestreckte Element ein Lumen aufweist, das zumindest teilweise durch den distalen Abschnitt angeordnet ist;
worin ein Endabschnitt der Schleife über eine Kanalverbindung oder eine Magnetverbindung mit dem Schaft verbunden ist; und
worin der distale Abschnitt des Führungsdrahts in das erste langgestreckte Element zurückgezogen werden kann, indem das distale Ende des langgestreckten Schafts mit einem Rand des ersten langgestreckten Elements in Berührung gebracht werden kann, um die Schleife zumindest teilweise von dem Schaft zu lösen.

10. Führungsdraht nach Anspruch 9, ferner umfassend eine Verbindungsregion zur lösbaren Verbindung des Schleifenabschnitts an dem Schaft.

11. Führungsdraht nach Anspruch 10, worin der Schaft einen Schaftablöseabschnitt umfasst, der in das erste langgestreckte Element zurückgezogen werden kann und wieder aus dem ersten langgestreckten Element ausgefahren werden kann, nachdem der Schleifenabschnitt von dem Schaft entfernt wurde.

12. Verfahren zum Lösen eines Schleifenabschnitts einer medizinischen Vorrichtung, wobei das Verfahren Folgendes umfasst:
Bereitstellung eines ersten langgestreckten Elements mit einem Lumen, das sich zumindest teilweise durch es hindurch erstreckt;
Bereitstellung eines zweiten langgestreckten Elements mit einem Schaft und einem distalen Abschnitt, der einen Schleifenabschnitt bildet, wobei sich der Schleifenabschnitt distal von dem zweiten langgestreckten Element erstreckt und das zweite langgestreckte Element einen proximalen Abschnitt aufweist, der sich zumindest teilweise durch das Lumen erstreckt,
Bereitstellung einer Kanalverbindung oder einer Magnetverbindung zur lösbaren Verbindung eines Endabschnitts der Schleife an dem Schaft zur Bildung einer Schleife;
Zurückziehen des zweiten langgestreckten Elements proximal durch das Lumen, um die Schleife zumindest teilweise von dem Schaft zu lösen.

13. Verfahren nach Anspruch 12, worin der distale Endabschnitt des zweiten langgestreckten Elements mit einem Rand des distalen Abschnitts des ersten langgestreckten Elements in Berührung gebracht wird, um die Schleife zu lösen.

## Revendications

1. Guide-fil (20) comprenant:
une tige allongée (22) présentant une partie proximale et une partie distale, la partie distale comportant une boucle (30) connectée de façon détachable à la tige en une première configuration bouclée, **caractérisé en ce que** la boucle peut être déconnectée en un point de séparation et forme une deuxième configuration dans laquelle la boucle est au moins partiellement déconnectée de la tige, et **en ce qu'**une partie d'extrémité de la boucle est connectée à la tige par une connexion en canal (32, 32A) ou une connexion magnétique (29, 34, 35).

2. Guide-fil selon la revendication 1, dans lequel la boucle est entièrement détachable de la tige dans la deuxième configuration.

3. Guide-fil selon la revendication 1 ou 2, dans lequel la partie distale comprend en outre une partie intermédiaire et une partie d'extrémité, la partie d'extrémité étant connectée de façon détachable à la partie intermédiaire pour former la boucle.

4. Guide-fil selon l'une quelconque des revendications précédentes, dans lequel au moins une partie du guide-fil comprend une matière bioabsorbable.

5. Guide-fil selon l'une quelconque des revendications précédentes, dans lequel au moins une partie du guide-fil contient du nitinol.

6. Guide-fil selon l'une quelconque des revendications précédentes, dans lequel au moins une partie du guide-fil comprend en outre un revêtement.

7. Guide-fil selon l'une quelconque des revendications précédentes, dans lequel la région de connexion comprend un matériau radio-opaque.

8. Guide-fil selon l'une quelconque des revendications précédentes, dans lequel le guide-fil est configuré en vue d'être disposé dans le tractus gastro-intestinal d'un patient.

9. Guide-fil selon l'une quelconque des revendications précédentes, en combinaison avec:
un premier élément allongé comportant une partie proximale et une partie distale, le premier élément allongé présentant une lumière disposée au moins partiellement à travers la partie distale;
dans lequel une partie d'extrémité de la boucle est connectée à la tige par une connexion en canal ou une connexion magnétique; et
dans lequel la partie distale du guide-fil est rétractable dans le premier élément allongé en mettant en contact l'extrémité distale de la tige allongée avec un bord du premier élément allongé afin de détacher au moins partiellement la boucle de la tige.

10. Guide-fil selon la revendication 9, comprenant en outre une région de connexion destinée à connecter de façon détachable la région de la boucle à la tige.

11. Guide-fil selon la revendication 10, dans lequel la tige comprend une partie de séparation de la tige qui est rétractable dans le premier élément allongé et ré-extensible à partir du premier élément allongé après que la boucle ait été détachée de la tige.

12. Procédé pour déconnecter une partie de boucle d'un instrument médical, le procédé comprenant les étapes suivantes:
procurer un premier élément allongé présentant une lumière s'étendant au moins partiellement à travers lui;
procurer un deuxième élément allongé comportant une tige et une partie d'extrémité distale formant une partie de boucle, la partie de boucle s'étendant de façon distale à partir du deuxième élément allongé et le deuxième élément allongé présentant une partie proximale s'étendant au moins partiellement à travers la lumière,
procurer une connexion en canal ou une connexion magnétique pour connecter de façon détachable une partie d'extrémité de la boucle à la tige pour former la boucle;
retirer le deuxième élément allongé de façon proximale à travers la lumière afin de déconnecter au moins partiellement la boucle de la tige.

13. Procédé selon la revendication 12, comprenant la mise en contact de la partie d'extrémité distale du deuxième élément allongé avec un bord de la partie distale du premier élément allongé afin de déconnecter la boucle.
